# EUROPEAN PATENT APPLICATION

(11) **EP 3 828 806 A1**
(43) Date of publication of application: **02.06.2021**
(21) Application number: 20795199.7
(22) Date of filing: 26.02.2020
(51) Int. Cl.: G06Q 50/26, G06F 21/32, H04L 9/32, A61B 5/117

(54) **METHOD AND DEVICE FOR AUTHENTICATING CARE ORGANIZATION BY AID ORGANIZATION TO PROTECT HUMAN RIGHTS OF PERSON TO BE TAKEN CARE OF**

(30) Priority: 25.04.2019 KR 20190048571
(71) Applicant: Oh, Stephen Sanggeun, Seoul 05788 (KR); Lee, Jin-Seo, Gangwon-do 24294 (KR)
(72) Inventor: Oh, Stephen Sanggeun, Seoul 05788 (KR); Lee, Jin-Seo, Gangwon-do 24294 (KR)
(74) Representative: Viering, Jentschura & Partner mbB Patent- und Rechtsanwälte
(86) International application number: PCT/KR2020/002771
(87) International publication number: WO 2020/218728

(57) **Abstract**

Disclosed is a method for authenticating a management entity by an aid entity to maintain human rights of a management object when the management entity applies to the aid entity a help of a protective management action for the management object which is difficult or impossible to do an independent living by himself/herself, the method comprising: an authentication module preparation step of registering original biometric information acquired from a biometric sensor in an authentication module of the management entity registered in an authentication server; a help application step of transmitting help application information of a protective management action for the management object to a reception server of the aid entity from a management terminal of the management entity; a biometric information input step of acquiring instantaneous biometric information from the biometric sensor and inputting the acquired instantaneous biometric information to the authentication module; a module determination processing step of determining a coincidence between the original biometric information and the instantaneous biometric information in the authentication module and transmitting a biometric coincidence information to the authentication server when the coincidence is within a predetermined range; an authentication confirmation step of transmitting the authentication confirmation information from the authentication server receiving the biometric coincidence information to a confirmation server of the aid entity; and an action step of completing the reception of the help application information by the confirmation server receiving the authentication confirmation information and generating an action command for the help by the aid entity.

## Description

### CROSS-REFERENCE TO RELATED APPLICATION

This application claims under 35 U.S.C. §119(a) the benefit of Korean Patent Application No. 10-2019-0048571 filed on April 25, 2019, the entire contents of which are incorporated herein by reference.

### BACKGROUND

### (a) Technical Field

The present invention relates to an aid entity's authentication method and apparatus for a management entity for protecting human rights of a management object, and more particularly, to a method and an apparatus by which an aid entity may authenticate a management entity while maintaining human rights of a management object when the management entity applies to the aid entity a help of a protective management action for the management object who is difficult or impossible to do an independent living by himself/herself.

### (b) Background Art

Generally, human rights refer to rights to pursue happiness while maintaining human dignity and values. In addition, guarantee of human rights means that the nation has the duty to identify and guarantee the inviolable basic human rights of individuals.

There are management objects which are difficult or impossible to do an independent living by themselves. Among human beings, for example, children, people with retardation, dementia, etc. have human dignity and values, but correspond to management objects which are difficult to do a living by themselves and are not easily subjected to protection of human rights by themselves.

Further, the number of pets traded in the pet market in Korea has already reached beyond 10 million. Many families now have at least one pet, and these pets are considered as the family of humans. However, like children, people with retardation, and dementia, the pets cannot do an independent living alone to become management objects.

With respect to these management objects, the help of other beings is required and it is preferable to protect the management objects by managing or helping some or most of life. The being of doing the help or protective management action for the management objects refers to a management entity. However, the management entity may be an individual such as a parent, a guardian, a manager, an owner, a doctor, or the like, or an organization such as a management company or a management association.

For example, a child (management object) needs to constantly receive the protective management action by a parent (management entity).

On the other hand, even when the management entity protects and manages the management object, there may be a case in which a third party's help for the management object is required. For example, when the management object (child) leaves the management area of the management entity (parent) due to missing, runaway, etc., a search of a police officer or the like is required. As another example, during the medical operation of the management object (dementia), a management entity (family) needs to request an operation requiring expertise to a doctor or the like. To sum up, the third party's help may be required out of a capacity of the management entity.

When the beings which are difficult or impossible to do an independent living by themselves, such as children, people with retardation, and dementia, run away or deviate from the protection range (house, facilities, etc.) of the management entity, the reality is that significant social costs of the nation, public institutions, etc. as well as the management entity are required. It is also very common for pets to deviate from the protection range (house, facilities, etc.) of the management entity, and during such deviation, for example, runaways, there may also occur damages such that pets are starved to death, sick or road-killed, while there may often occur cases such that pets cause problems of attacking humans or damaging crops.

As such, an individual or organization for helping the protective management action of the management entity for the management object is called an aid entity.

Such an aid entity may include public institutions formed by national and public organizations such as the nation, and private institutions formed by individuals, private companies, and private organizations. Personal aid entity may include doctors, bodyguards, sign language workers, and the like, and organization aid entity may include protection agencies, management companies, security companies, national agencies such as police and fire stations, and the like.

Even in the case of the aid entity, arresting or directly monitoring children, people with retardation, and dementia who have difficulty in asking for their own consent for protection corresponds to infringement of basic human rights and thus it is prohibited. Therefore, when the help of the aid entity for the management object is required, the management entity applies the help to the aid entity. The help may also be applied directly face-to-face, but may also be applied through telephones or information processing devices (a computer, a smartphone, etc.).

When the help is applied, the aid entity needs to authenticate whether the applicant is a legitimate management entity to prevent infringement of human rights for the management object. If the aid entity takes a helping action even when the application is not a legitimate application, the interests of the management object may be infringed by those with poor intentions, and the human rights of the management object may be breached.

In order to determine whether the applicant is a legitimate management entity, the aid entity may have in advance data to be used when identifying the management entity for each management object.

In Korean Patent Laid-open Publication No. 10-2006-0012729, there is disclosed 'a public certificate service system for a protected person comprising: a public certificate means which transmits contents related to a settlement price limit, protector's contact information, and a settlement method registered in a protector's certificate to an application server through a public packet network or a dedicated line, when a purchase history of the protected person purchased through a shopping mall server exceeds the payment amount limit allowed by the protector; the application server for extracting the protector's contact information from the contents transmitted by the public certificate means to connect a call to the corresponding contact through a service gateway; the service gateway which controls an actual network according to a command of an application program of the application server; and an intelligent peripheral device which is equipped with a DTMF digit collection/TTS(Text-To-Speech) function'. According to this, although there is disclosed a configuration in which the protector specifies the payment limit in advance, the process of verifying whether the protector is a legitimate guardian at the time of application of the payment limit is not disclosed.

### SUMMARY OF THE DISCLOSURE

No one, including national and public institutions, should infringe human rights in the name of protection. In other words, no one should infringe basic human rights by detaining or monitoring children, people with retardation, dementia, etc. Also, no one should abuse or misuse pets. Therefore, the aid entity should not directly manage the management object, but it is important that the identity of the management entity should be verified by the aid entity which receives the help application of the protection action for the management object.

In the related art, when the management entity applies a help for the protective management action of the management object to the aid entity, the aid entity requests a presence of a public certificate such as a resident registration card in face-to-face application, and compares a resident registration number and a photograph on the resident registration card manually with those of an applicant to determine whether the management entity is legitimate. In this case, if another person possesses and submits a resident registration card, it may be difficult to identify the resident registration card quickly and accurately, and since there are many people who have similar faces, in particular, there is a problem that there is a high possibility of error in determining the management entity from another person by only a photograph.

In addition, when the help is applied by a telephone, the management entity is determined by a telephone number registered in advance. However, it is difficult to determine whether the management entity is legitimate by only a voice on the telephone. In particular, it is impossible to discriminate the legitimate management entity when another person impersonates a management entity. In some cases, registered personal information such as some part of addresses or resident registration numbers may be queried and the answers thereof may be checked and supplemented, but it is difficult to determine the legitimate management entity when another person has seized important information in advance with respect to a method of checking the knowledge.

When the help is applied by an information processing device such as a computer or a smartphone, it is determined whether the management entity is legitimate by an ID/password with reference to a connected IP address. However, if the ID/password is leaked and another person inputs and logs-in instead, there is a problem that it is impossible to determine whether the management entity is legitimate or not.

In addition, the aid entity may be able to determine whether the management entity is legitimate through biometric authentication such as fingerprints, voiceprints, and irises. However, in the related art, original biometric information is configured to be stored in a computer network or an external server of the aid entity, and there is a concern that infringement of human rights of the management object may occur when a leakage accident of the stored biometric information occurs. In particular, in the configuration in which the biometric information is stored in the external server, there is a concern that the infringement of human rights of the management object may occur when a hacking occurs in a process of sending the biometric information input to a terminal to the external server for comparison purposes.

In order to solve the problems of the related art, an object of the present invention is to provide aid entity's authentication method and apparatus for a management entity for protecting human rights of a management object capable of protecting human rights of the management object by preventing the leakage of information about the management object and the management entity, without determining whether the management entity is legitimate by contrasting photographs manually, checking a knowledge of registered information on a telephone, or verifying an ID/password, storing original biometric information in a computer network or an external server of the aid entity, and transmitting biometric information itself in comparison with the input biometric information.

An aspect of the present invention to achieve the objects provides a method for authenticating a management entity by an aid entity to maintain protection of human rights of the management object when the management entity applies to the aid entity a help of a protective management action for the management object which is difficult or impossible to do an independent living by himself/herself, the method comprising: an authentication module preparation step of registering original biometric information acquired from a biometric sensor in an authentication module of the management entity registered in an authentication server; a help application step of transmitting help application information of a protective management action for the management object to a reception server of the aid entity from a management terminal of the management entity; a biometric information input step of acquiring instantaneous biometric information from the biometric sensor and inputting the acquired instantaneous biometric information to the authentication module; a module determination processing step of determining a coincidence between the original biometric information and the instantaneous biometric information in the authentication module and transmitting the biometric coincidence information to the authentication server when the coincidence is within a predetermined range; an authentication confirmation step of transmitting the authentication confirmation information from the authentication server receiving the biometric coincidence information to a confirmation server of the aid entity; and an action step of completing the reception of the help application information by the confirmation server receiving the authentication confirmation information and generating an action command for the help by the aid entity.

The authentication module may be implemented as wired or wireless hardware which is detachably connected to the management terminal.

The authentication module may be implemented to be included in the management terminal as software or hardware.

The biometric sensor may be implemented to be included in the authentication module or the management terminal as hardware.

The confirmation server may be implemented to be included in the reception server as software or hardware.

A sensor may be further included to check a normal existence range in which the management object is subject to be located, wherein the sensor may be configured to transmit a departure signal to the management terminal when the management object is out of the normal existence range.

The management terminal receiving the departure signal may be configured to output a warning signal.

The management terminal receiving the departure signal may be configured to automatically transmit the help application information to the reception server.

Encrypted management object information for the management object included in the help application information may be configured to be decrypted after receiving the authentication confirmation information.

Alternatively, after receiving the authentication confirmation information, the encrypted management object information which has been pre-stored in the reception server or the confirmation server may be configured to be decrypted.

Another aspect of the present invention to achieve the objects provides an apparatus for authenticating a management entity by an aid entity to maintain protection of human rights of a management object when the management entity applies to the aid entity a help of a protective management action for the management object which is difficult or impossible to do an independent living by himself/herself, the apparatus comprising an authentication server configured to transmit authentication confirmation information to a confirmation server of the aid entity when receiving biometric coincidence information from the authentication module after registering the authentication module of the management entity; a biometric sensor configured to acquire original biometric information to transmit the acquired original biometric information to the authentication module and acquire instantaneous biometric information at the time of help application to transmit the acquired instantaneous biometric information to the authentication module; an authentication module configured to receive and register the original biometric information from the biometric sensor in advance, and then receive the instantaneous biometric information from the biometric sensor at the time of help application to determine a coincidence with the original biometric information, and transmit the biometric coincidence information to the authentication server when the coincidence is within a predetermined range; a management terminal configured to transmit help application information of a protective management action for the management object to a reception sever of the aid entity; a reception sever configured to initiate an authentication procedure of the authentication module by receiving the help application information from the management terminal; and a confirmation server configured to complete a reception of the help application information by receiving the authentication confirmation information from the authentication server and generate an action command for the help of the aid entity.

Further, the present invention to achieve the objects provides a program recorded on a storage medium which is readable by an information processing device recording a program for executing each step of the method.

According to the present invention, there are provided aid entity's authentication method and apparatus for a management entity for protecting human rights of a management object capable of protecting human rights of the management object by preventing the leakage of information about the management object and the management entity, without determining whether the management entity is legitimate by contrasting photographs manually, checking a knowledge of registered information on a telephone, or verifying an ID/password, storing original biometric information in a computer network or an external server of the aid entity, and transmitting biometric information itself in comparison with the input biometric information.

### BRIEF DESCRIPTION OF THE DRAWINGS

The above and other features of the present invention will now be described in detail with reference to certain embodiments thereof illustrated in the accompanying drawings which are given hereinbelow by way of illustration only, and thus are not limitative of the present invention, and wherein:

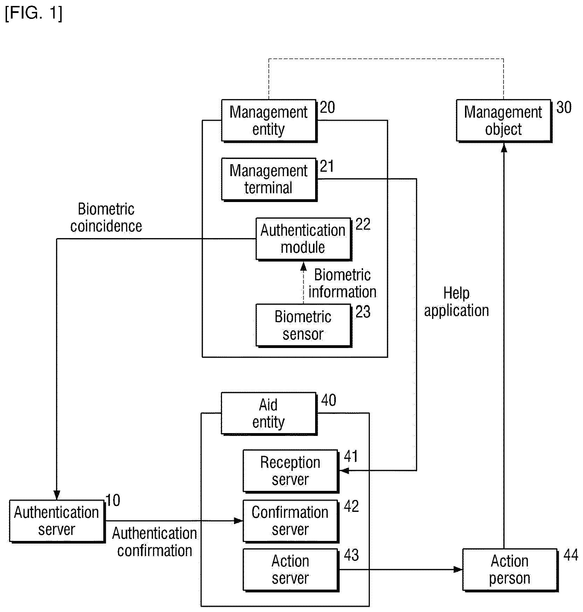

FIG. 1 is a block diagram of an embodiment of an apparatus in which an aid entity's authentication method for a management entity for protecting human rights of a management object of the present invention is implemented; and

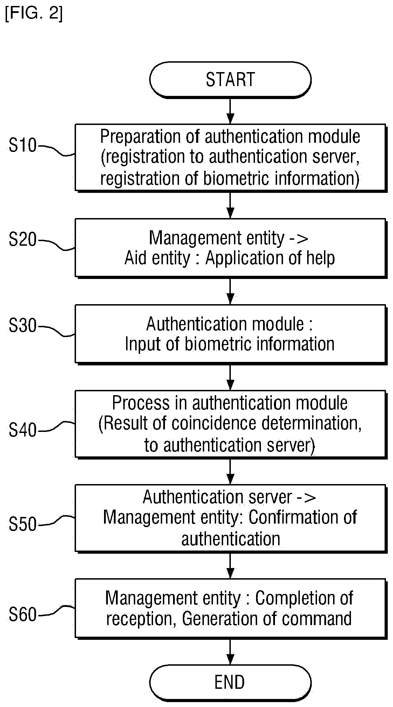

FIG. 2 is a flowchart of an embodiment of an aid entity's authentication method for a management entity for protecting human rights of a management object of the present invention.

### DETAILED DESCRIPTION

Hereinafter, aid entity's authentication method and apparatus for a management entity for protecting human rights of a management object according to the present invention will be described in detail with reference to the accompanying drawings. However, with respect to members having the same function by the same configuration, the detailed description may be omitted by maintaining the same reference numerals even if the drawings are varied.

In addition, a relationship in which a member is arranged or connected to front, rear, left, right, upper, or lower side of another member includes a case where a separate member is interposed therebetween. On the contrary, when a member is disposed 'directly' on front, rear, left, right, upper, or lower side of another member, it is meant that there is no separate member therebetween. In addition, when any part "comprises" other components, unless otherwise stated, it means that further other components may be included without excluding the same.

In the following description, dividing names of components into first, second, and the like is to divide the names because the components are the same as each other, and an order thereof is not particularly limited. In addition, the terms 'unit', 'means', 'part', 'member' or the like, which are described in the specification, mean units of a comprehensive configuration that performs at least one function or operation. In addition, an information processing device such as a terminal, a server, etc. described in the specification basically means hard wiring, which means hardware in which a specific function or operation is implemented, but should not be construed to be limited to specific hardware, and does not exclude soft wiring consisting of software that is driven so that the specific function or operation is implemented on general purpose hardware. That is, the terminal or server may be a device or may also be software installed on any device, such as an application.

In addition, since the size and thickness of each configuration illustrated in the drawings are arbitrarily shown for convenience of description, the present invention is not necessarily limited to those illustrated in the drawings, and in order to clearly express various parts and areas, such as layers and areas, the thickness, and the like may be exaggerated to be expanded or reduced.

### <Basic Configuration>

The present invention is an invention applied to the case where a management entity 20 applies to an aid entity 40 a help of a protective management action for a management object which is difficult or impossible to do an independent living by himself/herself. For example, the present invention may be applied to a case where a parent (management entity) reports a missing person to a police station (aid entity) when a child (management object) is missing. The management entity 20, the management object 30, and the aid entity 40 may be individuals or groups, and may be private or public facilities.

In particular, the present invention is a method for allowing the aid entity 40 to authenticate the management entity 20 while maintaining the protection of human rights of the management object 30. For example, when a parent (management entity) reports a missing person to a police station (aid entity) for a missing child (management object), if an unrelated third party or a malicious wrong third party is left alone to report the missing with contents different from the fact, the child's interests (property, life, honor, etc.) may be harmed and human rights may be infringed. The present invention allows a legitimate and true parent (management entity) to report the missing to the police station (aid entity) to protect the human rights of the child (management object). The management object 30 may include children, people with retardation, dementia, pets, and the like. The management entity 20 may include an individual such as a parent, a guardian, a manager, an owner, a doctor, or the like, or an organization such as a management company or a management organization. The aid entity 40 may include personal aid entity such as doctors, bodyguards, and sign language workers, and organization (group) aid entity such as protection agencies, management companies, security companies, and national agencies (police station, fire station, etc.).

The method of the present invention includes an authentication module preparation step S10; a help application step S20; a biometric information input step S30; a module determination processing step S40; an authentication confirmation step S50; and an action step S60.

The authentication module preparation step S10 is a step of registering original biometric information acquired from a biometric sensor 23 in an authentication module 22 of the management entity 20 registered in an authentication server 10.

The authentication server 10 is a server which transmits authentication confirmation information to a confirmation server 42 of the aid entity 40 when receiving biometric coincidence information from the authentication module 22 after registering the authentication module 22 of the management entity 20. The authentication server 10 may be provided outside the aid entity 40 in terms of professionalism, manageability, transparency, etc., but may be provided inside the aid entity 40 to improve security, flexibility and the like.

The authentication module 22 is a module which receives and registers the original biometric information from the biometric sensor 23, and then receives the instantaneous biometric information from the biometric sensor at the time of help application to determine a coincidence with the original biometric information, and transmits the biometric coincidence information to the authentication server 10 when the coincidence is within a predetermined range.

In order to register the biometric information, it is preferable that a secure memory (not illustrated) is provided inside the authentication module 22. This secure memory is a memory which denies any access from the outside and can be accessed only by a secure CPU. The secure CPU may be configured so that a booting program is recorded in a ROM at the factory, an authentication program is recorded in an SRAM, verification data for this authentication program is recorded in a special memory inside the CPU, the verification of the completeness of the authentication program is completed, and then the authentication program and the biometric data are read to the secure memory to determine the coincidence, and the resulting information is transmitted to the authentication server 10.

The authentication module 22 may be implemented as wired or wireless hardware, for example, a USB Dongle or a Bluetooth Dongle which is detachably connected to a management terminal 21 of the management entity 20, for example, a smartphone (management terminal) of a parent (management entity). Since the authentication module is detachably connected, portability is improved, and by being connected to another terminal, the terminal may be changed into another authentication terminal to be a terminal-independent authenticator.

The wire-connected hardware (e.g. sockets and plugs) may include, for example, a USB type (USB Dongle), but is not limited thereto, and may also use a standard for detachable hardware wired connection to the terminal, for example, a Micro 5-pin, an HDMI, a LAN interface, an SD card interface, a monitor interface, an earphone interface, a power interface, or the like. The wireless-connected hardware (e.g., radio wave) may be, for example, a Bluetooth type, but is not limited thereto, and may use a standard for detachable hardware wireless connection to the terminal, for example, WiFi, LoRa, infrared, optical communication, FM, AM, or the like.

On the other hand, the authentication module 22 may be implemented to be included in the management terminal 21 as software or hardware. As such, since the authentication module 22 is built in the management terminal 21, there is no risk of loss, and convenience is increased by managing the update and maintenance of the authentication module 22 together in the management terminal 21.

The biometric sensor 23 is a sensor that acquires original biometric information to transmit the acquired original biometric information to the authentication module 22, and acquires instantaneous biometric information at the time of help application to transmit the acquired instantaneous biometric information to the authentication module 22. The biometric information refers to bio data such as a fingerprint, a voiceprint, an iris, or the like. The original biometric information refers to biometric information as a reference for comparison. The instantaneous biometric information refers to biometric information to be newly input at the time of each help application. The comparison of both of biometric information is performed in the authentication module 22.

The biometric sensor 23 may be implemented to be included in the authentication module 22 or the management terminal 21 as hardware. As a result, it is convenient in that a function of the biometric sensor 23 may be managed together at the time of the maintenance and update of the authentication module 22 or the management terminal 21.

The help application step S20 is a step of transmitting a protective management action, e.g., help application information for missing search, such as a search team dispatch request for the management object 30, for example, a child to a reception server 41 of the aid entity 40 from the management terminal 21 of the management entity 20, for example, a smartphone (management terminal) of the parent (management entity).

The management terminal 21 is a terminal that transmits the help application information of the protective management action for the management object 30 to the reception server 41 of the aid entity 40.

The reception server 41 is a server that initiates an authentication procedure of the authentication module 22 by receiving the help application information from the management terminal 21. At the time of initiating the authentication procedure, the reception server 41 may send an authentication request code having uniqueness to the management terminal 21.

Such a help application may be performed through data communication between the information processing devices such as the management terminal 21 and the reception server 41. However, after a telephone call is first made, data for the information processing device may be transmitted and received by data communication through the telephone line. By the help application, that is, the transmission of the help application information, the authentication procedure of the authentication module 22 is initiated.

The biometric information input step S30 is a step of acquiring instantaneous biometric information from the biometric sensor 23 and inputting the acquired instantaneous biometric information to the authentication module 22.

In order to authenticate a legitimate management entity at the time of the help application, it is necessary to prove that a subject (entity) currently applying the help is a person having the same biometric information as the original biometric information which has been registered (stored) in the authentication module registered in the authentication server. Accordingly, the management entity 20 inputs biometric information, that is, instantaneous biometric information, to the authentication module 22 for comparison with the original biometric information by using the biometric sensor 23.

The module determination processing step S40 is a step of determining a coincidence between the original biometric information and the instantaneous biometric information in the authentication module 22 and transmitting the biometric coincidence information to the authentication server 10 when the coincidence is within a predetermined range.

The original biometric information which has been registered and encrypted in the secure memory in the authentication module 22 is compared with the instantaneous biometric information after decryption in a memory that cannot be externally accessed. As the comparison result, when the coincidence is equal to or greater than a predetermined value, the biometric coincidence information is transmitted to the authentication server 10 as a signal and otherwise, a predetermined error signal is transmitted. In any case, both the decrypted original biometric information and the instantaneous biometric information are discarded, and all intermediate data which have been generated during the coincidence verification process are discarded. Therefore, the biometric information is not accessible from the outside and all discarded, so that no hacking can occur at all.

When the authentication module 22 sends the comparison result to the authentication server 10, the authentication module 22 may send an authentication request code or corresponding information received from the reception server 41.

The authentication confirmation step S50 is a step of transmitting the authentication confirmation information from the authentication server 10 receiving the biometric coincidence information to the confirmation server 42 of the aid entity 40.

The confirmation server 42 is a server that completes the reception of the help application information by receiving the authentication confirmation information from the authentication server 10, and generates an action command for the help of the aid entity 40. The confirmation server 42 may receive and confirm the authentication request code or the corresponding information which has been issued by the reception server 41 when receiving the authentication confirmation information from the authentication server 10.

However, the confirmation server 42 may be implemented to be included in the reception server 41 as software or hardware. In this way, the confirmation server 42 is built in the reception server 41, so that the maintenance and update are performed together, thereby increasing convenience in management.

The action step S60 is a step of completing the reception of the help application information by the confirmation server 41 receiving the authentication confirmation information and generating an action command for the help by the aid entity 40. By the action command, for example, an action person 44, for example, a maneuver may be dispatched to the management object 30, for example, a missing child.

### <Effects>

By such a configuration, when the management entity applies the help of the protective management action for the management object to the aid entity, it is possible to prevent fraudulent application by forgery or deprivation of personal information knowledge by third parties other than the management entity, which was not possible in a manual visual contrast using a resident registration number or a photograph on a public certificate at the time of face-to-face application, confirmation by a phone number at the time of telephone application, or confirmation by an ID/password at the time of application by the information processing device. And by the configuration, it is possible to prevent the risk of hacking by not transmitting the biometric information to perform the comparison of the biometric information in the server, which was transmitted in a conventional biometric information authentication technology. In addition, according to the present invention, when a legitimate management entity (for example, a parent) makes a help application, the authentication is promptly and accurately performed, so that there becomes no fear of causing the infringement of human rights, such that the aid entity inquires personal information in a database regarding a management object (for example, a child).

In the present invention, the original biometric information is stored in the authentication module in an encrypted state and an external access prevention state, the instantaneous biometric information at the time of the help application is also loaded into an external access-prevented memory in the authentication module to be compared with the decrypted original biometric information. The authentication program used for comparison may be configured to operate in the secure memory after confirming that there is no hacking forgery, and to remove all intermediate data generated in the comparison process while leaving only the comparison result. The comparison result may be configured to be transmitted to the authentication server as signal information other than the biometric information. Therefore, the external leakage or the hacking of the transmission process of the biometric information cannot be generated at all to protect the biometric information of the management entity, thereby preventing the infringement of human rights and the infringement of profits of the management object.

### <Configuration with Sensor>

Meanwhile, a sensor for checking a normal existence range in which the management object is subject to be located may be further included. In this case, it is preferable that the sensor is configured to transmit a departure signal to the management terminal when the management object is out of the normal existence range.

The sensor may be made of a sensor using a radio such as infrared, laser, optical communication, ultrasonic, electromagnetic, Bluetooth, WiFi, and LoRa, and may be made of a visual sensor for tracking an object by image processing through a camera, an acoustic sensor for tracking an object by sound waveform analysis through a microphone, or the like.

In addition, in order to more clearly detect whether the management object is located within the normal existence range, a terminal possessed by the management object may be further provided. By tracking a sensor position by the terminal or tracking a terminal position by the sensor, it is possible to detect whether the management object is located in the normal existence range.

For example, when a child having a terminal is out of a radius of 500 meters from a LoRa transmitter installed at home, the LoRa transmitter may send a departure signal to the management terminal.

In addition, in a configuration with the sensor, the management terminal receiving the departure signal may be configured to output a warning signal. By this warning signal, the management entity may recognize the fact that the management object is out of a safety range. Based on this recognition, the management entity may send the help application information to the aid entity.

The management terminal receiving the departure signal may be configured to automatically transmit the help application information to the reception server. In other words, the management terminal may include an automatic reception module configured by software to transmit the help application information to the reception server. As a result, automatic reception is possible in response to an urgent situation, so that quick response is possible, and when the management entity does not recognize a warning signal of the management terminal by the sensor, it is very preferable in that a critical situation may be reported automatically.

### <Encrypted Management Object Information>

Meanwhile, it is preferred that the encrypted management object information for the management object included in the help application information is configured to be decrypted after receiving the authentication confirmation information.

As a result, there is no fear of hacking because the aid entity does not have management object information. In addition, since the management object information to be transmitted is also transmitted in an encrypted state, the management object information becomes relatively stable at the moment of hacking.

Alternatively, it is preferred that the encrypted management object information which has been pre-stored in the reception server or the confirmation server is configured to be decrypted after receiving the authentication confirmation information.

As a result, since the aid entity is provided with the management object information in advance, there is no fear of hacking in the transmission process. However, even if the information is leaked by hacking of the database of the aid entity, the information is encrypted so as to be relatively safe.

In addition, it is preferred that the management object information that has been encrypted as such is configured to be decrypted only in use and then automatically discarded after the use.

### <Apparatus>

An aid entity's authentication apparatus for a management entity for protecting human rights of a management object of the present invention is an apparatus for authenticating a management entity 20 by an aid entity 40 while the protection of human rights of a management object 30 is maintained when the management entity 20 applies to the aid entity 40 a help of a protective management action for the management object 30 who is difficult or impossible to do an independent living by himself/herself. The apparatus is configured to include an authentication server 10; a biometric sensor 23; an authentication module 22; a management terminal 21; a reception server 41; and a confirmation server 42.

The authentication server 10 is a server which transmits authentication confirmation information to the confirmation server 42 of the aid entity 40 when receiving biometric coincidence information from the authentication module 22 after registering the authentication module 22 of the management entity 20.

The biometric sensor 23 is a sensor that acquires original biometric information to transmit the acquired original biometric information to the authentication module 22, and acquires instantaneous biometric information at the time of help application to transmit the acquired instantaneous biometric information to the authentication module 22.

The authentication module 22 is a module which receives and registers the original biometric information from the biometric sensor 23, and then receives the instantaneous biometric information from the biometric sensor at the time of a help application to determine a coincidence with the original biometric information, and transmits the biometric coincidence information to the authentication server 10 when the coincidence is within a predetermined range.

The management terminal 21 is a terminal that transmits the help application information of the protective management action for the management object 30 to the reception server 41 of the aid entity 40.

The reception server 41 is a server that initiates an authentication procedure of the authentication module 22 by receiving the help application information from the management terminal 21.

The confirmation server 42 is a server that completes the reception of the help application information by receiving the authentication confirmation information from the authentication server 10, and generates an action command for the help of the aid entity 40.

### <Program>

A program of the present invention of the aid entity's authentication method for the management entity for protecting the human rights of the management object is a program recorded in a storage medium that is readable by an information processing device recording a program for executing each step of the method on the information processing device.

The present invention may be used in an industry related to aid entity's authentication method and apparatus for a management entity for protecting human rights of a management object.

While the preferred embodiments of the present invention have been described above, the present invention is not limited to the above-described embodiments. The present invention may be implemented by modifications in various forms within the scope of appended claims, the detailed description of the invention, and the accompanying drawings, and other equivalent embodiments are possible. It is natural to those skilled in the art that the modifications and equivalent embodiments belong to the scope of the present invention. And the embodiments are provided only to complete the disclosure of the present invention and to fully inform the scope of the present invention to those skilled in the art to which the present invention belongs, and the present invention is only defined by the scope of the appended claims.

## Claims

1. An aid entity's authentication method for a management entity for protecting human rights of a management object, as a method for authenticating the management entity by the aid entity to maintain human rights of the management object when the management entity applies to the aid entity a help of a protective management action for the management object which is difficult or impossible to do an independent living by himself/herself, the method comprising:
an authentication module preparation step of registering original biometric information acquired from a biometric sensor in an authentication module of the management entity registered in an authentication server;
a help application step of transmitting help application information of a protective management action for the management object to a reception server of the aid entity from a management terminal of the management entity;
a biometric information input step of acquiring instantaneous biometric information from the biometric sensor and inputting the acquired instantaneous biometric information to the authentication module in which the original biometric information is registered in advance;
a module determination processing step of determining a coincidence between the original biometric information and the instantaneous biometric information in the authentication module provided with the original biometric information and the instantaneous biometric information while the biometric information is not leaked or transmitted outside and transmitting a biometric coincidence information to the authentication server when the coincidence is within a predetermined value range;
an authentication confirmation step of transmitting an authentication confirmation information from the authentication server receiving the biometric coincidence information to a confirmation server of the aid entity; and
an action step of completing the reception of the help application information by the confirmation server receiving the authentication confirmation information and generating an action command for the help by the aid entity,
wherein the biometric sensor is implemented to be included in the authentication module or the management terminal as hardware.

2. The method of claim 1, wherein the authentication module is implemented as wired or wireless hardware which is detachably connected to the management terminal.

3. The method of claim 1, wherein the authentication module is implemented to be included in the management terminal as software or hardware.

4. The method of any one of claims 1 to 3, wherein the confirmation server is implemented to be included in the reception server as software or hardware.

5. The method of any one of claims 1 to 3, wherein a sensor is further included to check a normal existence range in which the management object is subject to be located, the sensor being configured to transmit a departure signal to the management terminal when the management object is out of the normal existence range.

6. The method of claim 5, wherein the management terminal receiving the departure signal is configured to output a warning signal.

7. The method of claim 5, wherein the management terminal receiving the departure signal is configured to automatically transmit the help application information to the reception server.

8. The method of claim 1, wherein the encrypted management object information for the management object included in the help application information is configured to be decrypted after receiving the authentication confirmation information.

9. The method of claim 1, wherein the encrypted management object information which has been pre-stored in the reception server or the confirmation server is configured to be decrypted after receiving the authentication confirmation information.

10. An aid entity's authentication apparatus for a management entity for protecting human rights of a management object, as an apparatus for authenticating the management entity by the aid entity to maintain human rights of the management object when the management entity applies to the aid entity a help of a protective management action for the management object which is difficult or impossible to do an independent living by himself/herself, the apparatus comprising:
an authentication server configured to transmit authentication confirmation information to a confirmation server of the aid entity when receiving biometric coincidence information from the authentication module after registering the authentication module of the management entity;
a biometric sensor configured to acquire original biometric information to transmit the acquired original biometric information to the authentication module and acquire instantaneous biometric information at the time of help application to transmit the acquired instantaneous biometric information to the authentication module in which the original biometric information is included in advance;
an authentication module configured to receive and register the original biometric information from the biometric sensor in advance, and then receive the instantaneous biometric information from the biometric sensor at the time of help application to determine a coincidence between the original biometric information which has been registered in advance and the instantaneous biometric information inputted at the time of the help application in the authentication module while the biometric information is not leaked or transmitted outside, and transmit the biometric coincidence information to the authentication server when the coincidence is within a predetermined value range;
a management terminal configured to transmit help application information of a protective management action for the management object to a reception sever of the aid entity;
a reception sever configured to initiate an authentication procedure of the authentication module by receiving the help application information from the management terminal; and
a confirmation server configured to complete a reception of the help application information by receiving the authentication confirmation information from the authentication server and generate an action command for the help of the aid entity,
wherein the biometric sensor is implemented to be included in the authentication module or the management terminal as hardware.

11. A program recorded on a storage medium which is readable by an information processing device, the storage medium recording the program for executing each step of the method of any one of claims 1 to 3.
